# EUROPEAN PATENT APPLICATION

(11) **EP 3 190 170 A1**
(43) Date of publication of application: **12.07.2017**
(21) Application number: 17153893.7
(22) Date of filing: 30.09.2016
(51) Int. Cl.: C12M 1/00, C12M 1/08, C12M 1/34

(54) **BIOREACTOR WITH SEPARATE CO2 SUPPLY**

(30) Priority: 30.09.2015 GB 201517290; 23.10.2015 GB 201518810
(62) Divisional of application: 16778287.9
(71) Applicant: SUBITEC GMBH, 70597 Stuttgart (DE)
(72) Inventor: BERGMANN, Peter, 70597 Stuttgart (DE); BEYER, Lars, 70597 Stuttgart (DE); TRÖSCH, Walter, 70597 Stuttgart (DE); RUPPEL, Valentina, 70597 Stuttgart (DE); RIPPLINGER, Peter, 70597 Stuttgart (DE)
(74) Representative: Harrison, Robert John

(57) **Abstract**

A bioreactor is disclosed. The bioreactor comprises at least one rise chamber containing a growth medium and at least one down chamber connected to the at least one rise chamber at a top and at a bottom to form a loop. A first gas inlet is connected to the at least one rise chamber for supplying a first gas flow to the at least one rise chamber. A second gas inlet is connected in the loop to supply a second gas flow to the loop, the second gas flow being enriched, compared to air, with carbon dioxide

## Description

### FIELD OF THE INVENTION

The invention relates to a bioreactor for cultivating phototrophic microorganisms, such as but not limited to algae and cyanobacteria, as well as phototrophically grown cells, such as but not limited to moss. It furthermore relates to a method for operating such a bioreactor.

### BACKGROUND OF THE INVENTION

Bioreactors are fermenters in which a biological conversion of substances is carried out using, for example enzymes, microorganisms (bacteria, fungi, yeast, algae, cyanobacteria) as well as animal and plant cells, e.g. mosses. The operating conditions, such as temperature, pH value and nutrient condition can be adjusted within the bioreactor to optimize operating conditions for the conversion process. The function of the bioreactor includes therefore transfer of materials in a fluid phase (mixing), dispersion in a second phase - in most cases air - in order to obtain a large phase boundary surface for good material transfer from gas to liquid, and thermal transfer for temperature regulation. The design of a bioreactor depends on its applications and therefore must take into account the specific requirements of the biological system used.

In order to cultivate phototrophic microorganisms as well as phototrophically grown cells, so-called photobioreactors are used. Among potential designs, a so-called "airlift" photobioreactor can be used for cultivating phototrophic microorganisms and enabling the growth of the phototrophic microorganisms to a high cell density. Such airlift photobioreactors are produced by Subitec GmbH in Stuttgart.

The airlift loop photobioreactor has a column-shaped reactor vessel, in which introduction of air at the bottom of a rise chamber creates a fluid circulation of the growth medium within a loop defined by a shape of the photobioreactor. The airlift loop bioreactor is divided thus into a gassed zone (rise chamber) and an ungassed zone (down chamber). The gassed zone and the ungassed zone are interconnected at their bottom and their top so that the hydrostatic pressure differential causes a pump effect that results in an overflow of the fluid containing the growth medium from the gassed zone. The mixing of the growth medium in the reactor is due substantially to the aeration volume per time and thus this photobioreactor design enables a good mixing and a high gas-fluid mass exchange with low energy consumption. Such an airlift photobioreactor is described, for example, in the patent documents DE 199 16 597 or GB 2 235 210.

Another example of a photobioreactor is disclosed in US Patent No. 7,374,928 B2 (Trösch), which discloses the presence of transversal septa (or dividing walls) arranged cross-wise to the airlift flow and constrict the airflow to form a meandering path (only the side view shows a meander)- within the photobioreactor. This meandering gas flow creates a turbulent liquid flow and thus promotes mixing of the phototrophic microorganisms and the phototrophically grown cells within the photobioreactor and creates light dilution for individual ones of the phototrophic microorganisms and the phototrophically grown cells, when the light source is unidirectional.

Other examples of photobioreactors are known in the art. For example European Patent Application No EP 2 840 128 teaches a photobioreactor which comprises a tank having an external wall, an inlet for adding a medium for algae growth, an outlet for extracting algae from the photobioreactor. The photobioreactor includes a CO₂ applying means and lighting means. The tank has a first chamber and an intercommunicating second chamber, such that the medium of the tank can flow between the two chambers. The CO₂ applying means is arranged in the first chamber for supplying carbon dioxide to the medium and may also comprise means for applying a pressure wave. The pressure waves are described as "hitting" the carbon dioxide bubbles produced by the CO₂ applying means to increase the absorption of nutrients.

Chinese Utility Model CN 2015 93046 teaches also an air-lift circulating algae bioreactor. The bioreactor comprises a first pipe column and a second pipe column which are arranged at a distance from each other, an upper connecting pipe, a lower connecting pipe and an inflation unit. The first pipe column comprises a pipe wall defining a first incubating space, and the second pipe column comprises a pipe wall defining a second incubating space. An upper opening part and a lower opening part are formed at two opposite ends of each of the pipe walls. The upper and the lower connecting pipes are connected between the upper opening parts as well as between the lower opening parts, so as to communicate between the first and the second incubating spaces. The inflation unit can supply air or gas from an external gas source and provides a mechanism to force circulation between the first pipe column or the second pipe column, so that liquid filled therein can flow from one of the incubating spaces to the other incubating space to achieve the circulating flow effect.

US Patent Application Publication No US 2014/242681 teaches a photobioreactor system that may include one or more fluidly connectable tubes for growing or producing microorganisms or biomass, such as microalgae. The system may include either a single tube loop or an array of tubes. A first fluid may be held in the tube or tubes, and an inlet port may be provided for introducing a second fluid into the tube or tubes. The tube or tubes may be arranged vertically so that the second fluid rises through the tube or tubes. An outlet port may be provided at the top of the tube or tubes to remove the second fluid. The second fluid may be recirculated through the system via inlet and outlet lines as well as a pump and a replaceable reservoir for holding the second fluid. Where there is an array of tubes, a single inlet and outlet line may be sued for each tube.

International patent application No WO 2016/086165 teaches bioreactors that include a vessel with sides and a bottom. At least one opening in the vessel is connected to a means for introducing a gas, and at least one scaffold in the vessel is oriented substantially vertically in the vessel. The scaffolds are two substantially parallel sheets that are separated by a distance. Also disclosed are bioreactors that include a vessel with sides and a bottom, at least one opening in the vessel connected to a means for introducing a gas, and at least two scaffolds in the vessel oriented substantially vertically in the vessel. The patent application also includes methods of culturing cells including incubating a suspension of cells in a disclosed bioreactor and introducing a gas through the at least one opening in the vessel. In some examples, the cells include microalgae, macroalgae, bacteria, fungi, insect cells, plant cells, or animal cells.

Chinese Patent Application No 104328044 teaches a photobioreactor, which, during a photoautotrophic cultivation process, is used for adapting replenishment of carbon dioxide with light intensity by virtue of dynamic regulation of carbon dioxide by detecting changes of illumination intensity for photosynthesis and the concentration of liquid carbon dioxide in the system. The photobioreactor disclosed is used for effectively guaranteeing the coordination of energy and material input in the photosynthesis and is especially applicable to outdoor cultivation which uses natural light as a light source.

The prior art bioreactors all teach air or gas supply units which supply a continuous stream of gas to the bottom of one or more of the bioreactors to achieve an "airlift" effect that mixes the growth medium, as described in the US Patent No 7,374,928.

### BRIEF SUMMARY OF THE INVENTION

This disclosure teaches a bioreactor comprising at least one rise chamber containing a growth medium and at least one down chamber connected to the at least one rise chamber at a top and at a bottom to form a loop. A first gas inlet is connected to the at least one rise chamber for supplying a first gas flow to the at least one rise chamber. A second gas inlet 63 is connected in the loop to supply a second gas flow to the loop. The second gas flow is, compared to air, enriched with carbon dioxide.

The use of two separate gas inlets enables one of the gas inlets to be used to generate the flow of the growth medium throughout the bioreactor and to enable turbulence to be generated at least within the rise chamber to promote growth of the phototropic organisms within the bioreactor. The second gas inlet is used to supply carbon dioxide in a controlled manner into the bioreactor.

Typically the second gas flow is air and can be enriched to 1-10% with carbon dioxide. In the fall chamber, an even greater concentration of more than 10% can be supplied.

The second gas inlet can be located at the top of or in the at least one fall chamber. This enables a long path for the carbon dioxide to be absorbed by the growth medium.

The method of operation of a bioreactor comprises supplying the bioreactor with a growth medium for growing at least one of phototrophic microorganisms or phototrophically grown cells, supplying the bioreactor with a first gas flow to intermix the growth medium, the growth medium including at least one of the phototrophic microorganisms or the phototrophically grown cells, and a second gas flow which, compared to air, is enriched with carbon dioxide. The carbon dioxide can be supplied substantially bubble-free.

### BRIEF DESCRIPTION OF THE DRAWINGS

Fig. 1 shows a bioreactor.
Fig. 2 shows a side view in form of a rise chamber of the bioreactor.
Figs 3A and 3B show a series of experimental results illustrating the interruption of the gas flow.
Figs. 4A to 4C show the effect of light and flow rate on the growth.
Figs. 5A and 5B shows the effect of night and day on the growth.
Fig. 6 shows the dry weight of algae extracted from tbe bioreactor after a period of time.
Fig. 7 shows the productivity of the bioreactor

### DETAILED DESCRIPTION OF THE INVENTION

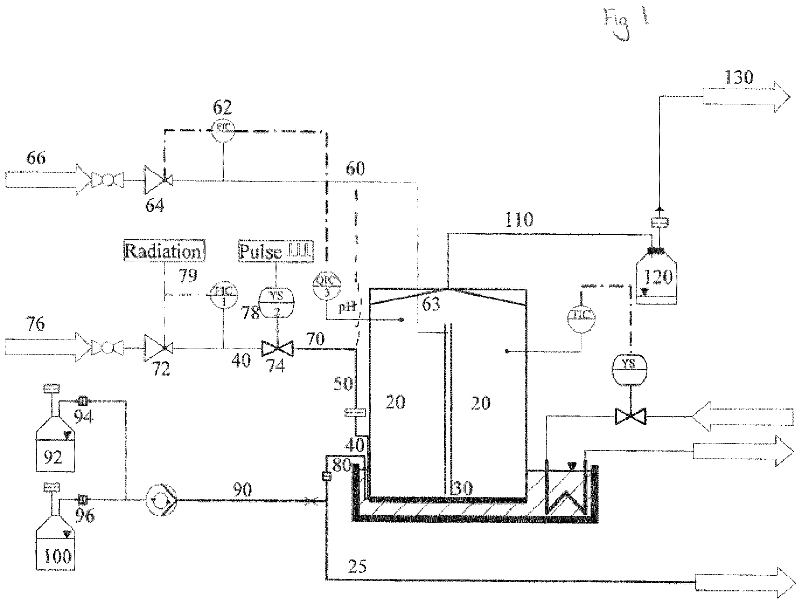

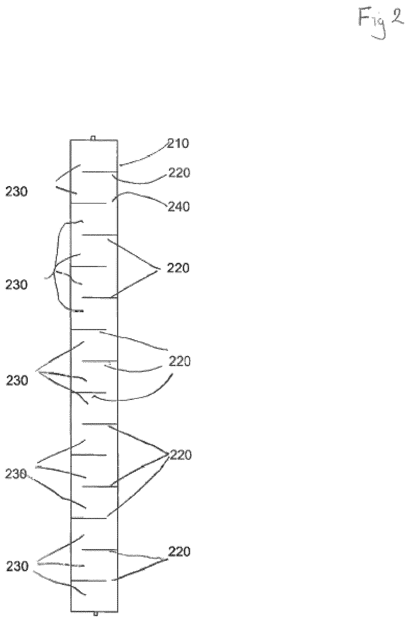

FIG. 1 shows a bioreactor 10 of the disclosure. The bioreactor 10 comprising two rise chambers 20 separated by a down chamber 30. The two rise chambers 20 and the down chamber 30 comprises a growth medium for growing phototrophic microorganisms, such as but not limited to algae or cyanobacteria, or phototrophically grown cells, such as mosses. Non-limiting examples of algae are the green algae *Chlorella sorokiniana* and the cyanobacterium Thermosynechococcus elongatus. The construction of the rise chambers 20 will be described in connection with Fig. 2: It will be appreciated that the bioreactor 10 of Fig. 1 has two rise chambers 20 and a single down chamber 30. Other bioreactors 10 may have a different number of rise chambers 20 and down chambers 30. The down chambers 30 can be arranged internally (as shown in Fig. 1) or external to the bioreactor 10.

The bioreactor 10 has a gas inlet 40, which is connected to the bottom of the rise chambers 20 and carries gas from the gas pipe 50 and provides the gas to the rise chambers 20 to form a gassed zone within the rise chambers 20. The bioreactor 10 has also a medium input 80 connected to a medium pipe 90 which carries medium into the bioreactor 10.

The gas pipe 50 can be connected to a carbon dioxide pipe 60, which carries carbon dioxide from a carbon dioxide source 66 through a carbon dioxide valve 64. The flow rate of the carbon dioxide in the carbon dioxide pipe is measured by the carbon dioxide flow meter 62. The connection between the gas pipe 50 and the carbon dioxide pipe 60 is shown as a dotted line in Fig. 1 because of an alternative connection, as outlined below.

The gas pipe 50 is further connected to an air pipe 70, which transports air from an air source, such as a compressor 76, through a mass flow controller 74 and/or a controllable magnetic air valve 72. The amount of air moving through the air pipe 70 can be controlled by a mass-flow controller 72. The magnetic air valve 72 and the mass flow controller 74 are connected to a controller 78, which measures and controls the flow of air through the air pipe 70, as will be described later. It will be appreciated that the magnetic air valve 72 could be replaced by other types of valves to open and close the air pipe 70 and thus control the movement of the air through the air pipe 70. It will also be appreciated that the air pipe 70 does not need both the magnetic air valve 72 and the mass flow controller 74. The mass flow controller 74 is particularly useful in connection with changing the flow of the air through the air pipe dependent on the amount of artificial light or sunlight falling on the bioreactor 10, as will be explained later.

The medium input 80 is connected to a medium pipe 90, which is connected through a first medium valve 94 to a first medium source 92 or through a second medium valve 96 to a second medium source 100. The first medium source 92 and the second medium source 100 have the various components of the growth medium for the biological material in the bioreactor 10.

The top of the bioreactor 10 has an outlet pipe 110 for removing excess gas through a filter 120 to an exhaust 130. The bioreactor 10 has at least one product outlet 25 to collect products produced in the bioreactor 10.

Fig. 2 shows an example of one of the rise chambers 20. The rise chamber has wall elements 210 made, in one aspect, of a thin, flexible, light-permeable plastic foil. The two wall elements 210 are constructed to have transverse elements 220 projecting from their inner sides. The transverse elements 220 are arranged parallel to each other and facing each other. In the displayed aspect of the bioreactor 10, there are seven transverse elements mounted on the inner sides of the wall elements 210. When the two wall elements 210 are placed together, as shown in Fig. 2, the transverse elements 220 form, in this example, fifteen interior chambers 230, divided by the transverse elements 220.

The transverse elements 220 do not completely close the interior of the rise chambers. There is still a plurality of small channels 240 formed between the inner wall of the wall elements 210 and the transverse elements 220. This plurality of small channels 240 allows passage of the medium and the gas from one end (bottom) of the rise chamber 20 through the interior chambers 230 to the top end of the rise chamber 20.

During operation of the bioreactor 10, the two rise chambers 20 are vertically positioned so that gas entering through the gas inlet 40 and the growth medium entering through the medium inlet 80 can rise through the small channels 240 and the interior chambers 230 of the rise chambers 20, as described in US Patent No US 7,374,928. The gas mixes the growth medium and supplies the growth medium with carbon dioxide. The plurality of transverse elements 220 thus form static mixers to enable mixing of the medium and the gas within the interior chambers 240. The gas is typically environmental air.

The gas is expelled through the outlet pipe 110. The growth medium is, as explained in US 7,374,928, allowed to cycle down through the down chamber 30 where it is collected and allowed to rise again through the rise chambers 20.

The purpose of the gas inlet 40 is to aerate the growth medium by creating bubbles in the rise chambers 20 to mix up the growth medium, and thereby to mix up the phototrophic cells in the growth medium, drive the growth medium and to add the carbon dioxide gas to the bioreactor 10 to enable growth. Typically, the air is enriched to 1-10% by volume with scrubbed carbon dioxide gas. Part of the carbon dioxide gas provided from the carbon dioxide source 66 will, however, be lost through the outlet pipe 110 with the expelled air. The aeration leads to elimination of any chemical concentration gradients in the bioreactor 10. It also addresses the effects of photolimitation and photoinhibition because of the growth of algae within the bioreactor 10 limiting the amount of light reaching the interior of the rise chambers 20 (so-called self-shadowing due to the increasing phototrophic cell numbers per unit volume).

It is also possible to install a separate carbon dioxide inlet 63 to the top of or inside the down chamber 30 to add carbon dioxide at this point in the bioreactor 10. This separate carbon dioxide inlet would allow separate of the mixing and turbulence in the interior chambers by aeration from provision of the carbon dioxide supply. The separate inlet provides furthermore more time for the carbon dioxide in gaseous form to be absorbed into the medium during the cycle as the carbon dioxide would have time to be absorbed whilst the medium was being cycled down the down chamber 30, as well as up through the rise chamber 20. The carbon dioxide supply can be implemented substantially bubble free.

The air valve 72 and/or the mass flow controller 74 control the flow of the air from the compressor 76 through the bioreactor 10. The air valve 72 and/or the mass flow controller 74 works in conjunction with the controller 78 and can provide either a continuous flow of air (including in one aspect of the invention the carbon dioxide gas from the carbon dioxide pipe 60) to the bottom of the bioreactor 10 through the gas inlet 40 (as known in US 7,374,928) or provide pulses of gas (including air and, if connected, carbon dioxide) by stopping and starting flow ("interrupting") of the gas and thus creating discrete pulses of gas and not just a continuous gas flow. The provision of air through the air pipe 70 to the air inlet 40 consumes a significant amount of energy. By using the air valve 72 to provide the discrete pulses of air to the rise chambers 20 of the bioreactor 10, the amount of energy consumed by the bioreactor can be significantly reduced.

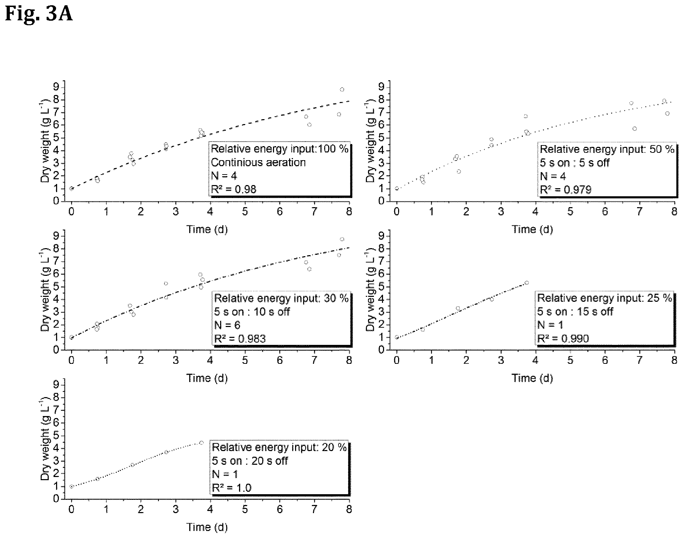

The graphs in Fig. 3A shows the effect of providing the interruptible pulses of the air to the rise chambers for a 281 bioreactor 10. The first graph shows the growth of the material in the bioreactor over time in day for continuous air flow. Four tests were carried out (N=4) and the curve fitted with a standard deviation R² of 0.98. The second graph shows a 1:1 pulse rate for the air flow (in this case 5s on and 5s off) in which it will be seen that there is no significant amount of loss of productivity, but an energy saving of 50%. The third graph shows a similar result for the case in which there is a 1:2 pulse rate, i.e. 5s of a discrete block of pulsed air followed by a pause of 10s. There is no significant loss of productivity, but only about 1/3 of the energy input.

The next two graphs show a loss of productivity, i.e. growth in the bioreactor 10 is substantially reduced and the air provided for turbulence is much smaller and not sufficient for optimal growth of the phototrophic cells. In both cases, the air was pulsed for five seconds and then turned off for 15 seconds or for 20 seconds. The experiments illustrated in Fig. 3A demonstrate that it is possible to reduce the amount of energy required to produce the air flow for optimal productivity for a given illumination.

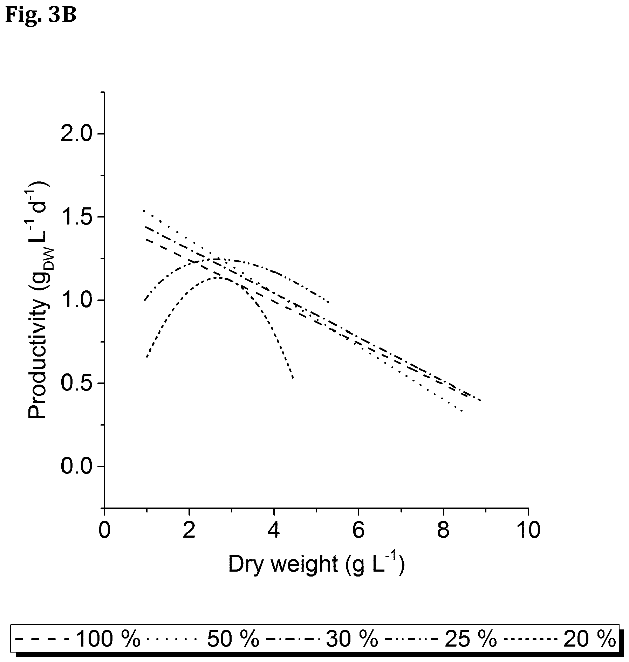

These results are summarized in Fig. 3B which shows on the vertical axis the productivity (dry weight per liter of growth medium per day) and the dry weight per liter on the horizontal axis for various pulse rates. It will be seen that there is no loss of productivity between continuous flow and a 1:2 pulse rate, but that the productivity drops off for lower pulse rates for a given illumination.

The length of time of the discrete air pulses depends on the size of the reactor. The results shown in Fig. 3B are based upon a 281 reactor. A 1801 reactor will require a longer pulse of air, but the relationship between the on and off of the discrete air pulses can be kept the same. Generally, it is thought that the discrete air pulse should be the same length as the time such that the air bubbles can rise from the bottom to the top through the rise chamber 20. It will be appreciated that the discrete air pulse leads to a formation of a series of air bubbles in the rise chamber 20.

In one aspect of the bioreactor 10, it is possible to adjust the air flow using the mass flow controller 74 in order to take into account the amount of light falling on the bioreactor 10 and detected by a PAR sensor 79. The PAR sensor 79 detects the photosynthetically active radiation (PAR) (similar but not identical to visible light) falling on the bioreactor 10. For example, on a cloudy day a significantly smaller amount of light, i.e. photosynthetically active radiation, will fall on the bioreactor 10, which means that the energy available for cultivation of phototrophic cells is reduced. This means that the amount of air required for mixing/dissipation the medium by aeration in the bioreactor 10 is reduced because of the lower light availability and slower growth. The controller 78 is connected to the PAR detector 79 and also to a flow meter and can measure and adjust the amount of air flowing through the air pipe 70 (and also the amount of carbon dioxide flowing through the carbon dioxide control valve 64 can be reduced). This will save energy and also reduce consumption of carbon dioxide by the bioreactor 10. For example, the amount of air flowing through the air pipe 70 will be adjusted to optimize the growth of the phototrophic cells in the bioreactor 10 dependent on the amount of artificial light or sunlight falling on the bioreactor 10.

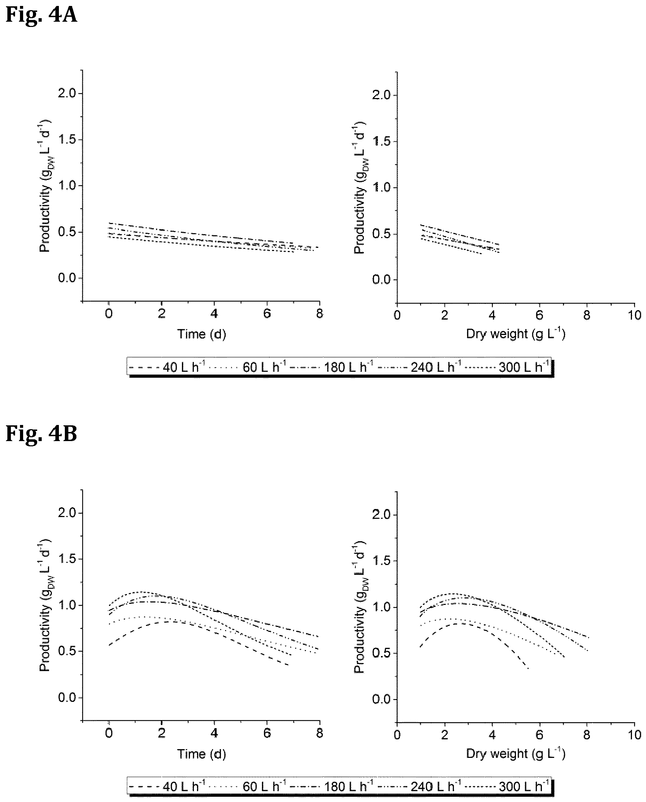

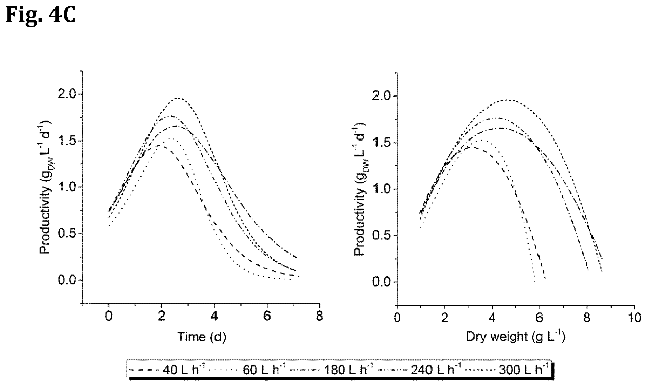

Figs 4A to 4C shows the effect of light on growth for various different differing flow rates of the air. Fig. 4A shows a light intensity of 180 µmol PAR photons / m². s; Fig. 4B shows a light intensity of 405 µmol photons PAR / m². s and Fig. 4C shows a light intensity of 780 µmol PAR photons / m².s. It will be seen that, for the lower intensity light of Fig. 4A, the differing flow rates of the air has almost no effect on the growth rate in the bioreactor 10. The fall-off in the growth rate is due to the increasing concentration of the grown materials in the bioreactor 10 preventing use of some of the light. Fig. 4B shows a slight increase in the growth rate with increased flow rate of the air and Fig. 4C shows a much larger increase in the growth rate with increase flow rate of the air. Thus, one can conclude that at lower light intensities, the flow rate of the air can be reduced to save on energy without substantially affecting the productivity.

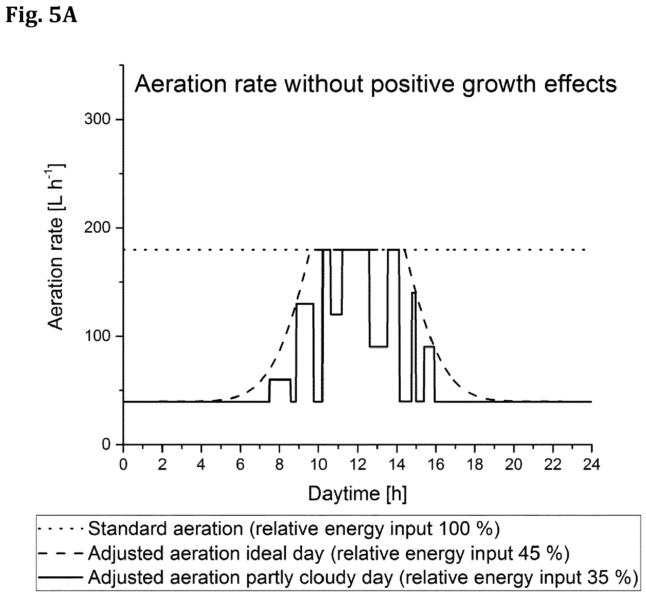

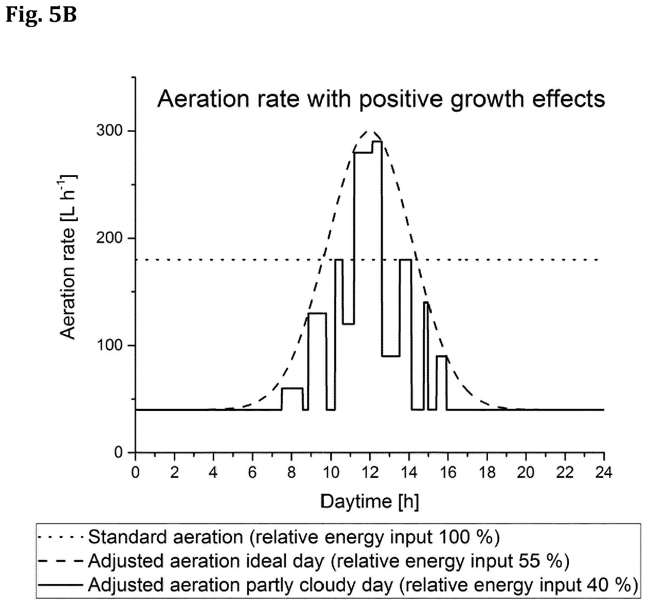

This can be put into practice as shown in Figs. 5A and B, which illustrate the effect of increasing the air flow when there is an increased amount of sunlight on an ideal, sunny day. It will be seen that maintaining the aeration rate at a constant value means that energy is wasted during the night hours or times of dim light (e.g. morning and evening hours) because there is a reduced rate of production due to less light. However, during the midday period the potential of the bioreactor 10 is not exploited. By increasing the amount of aeration during the midday period, the biological conversion would significantly increase.

Fig. 5B shows, for example, that the maximum aeration rate of the illustrated example is around 300 l/h but at night or during a clouded day it would be possible to use around 40 l/h with a large saving of energy.

### Examples

In one non-limiting example of the bioreactor 10, the growth medium in the bioreactor 10 comprised 300 mg/l of NH₄, 200 mg/l of PO₄ and 2,5 mg/L of iron citrate. The medium had a pH value of 6.6 to 7.5 The flow of the air was 360 l/hr. and the flow of the carbon dioxide was 20 l/hr. The light intensity was 516 µmol photons PAR /m²s.

In other non-limiting examples, the flow of the air could be between 20 and 400 1/hr. and the light intensity between 180 and 780 µmol photons PAR / m²s.

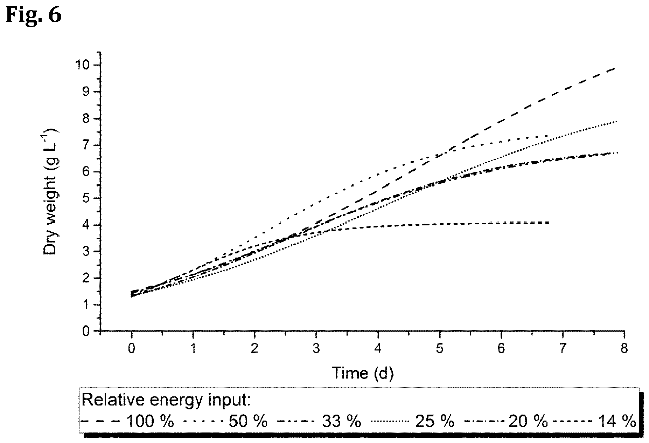

Fig. 6 shows a comparison of the dry weight of algae extracted after a period of time measured in days. The different curves show the input values. An input of 50% means, for example, that the ratio of discrete air pulses to no gas pulses is 1:1 and for 25% the ration is 1:3, i.e. 5 seconds of discrete air pulse, followed by 15 seconds of no pulse.

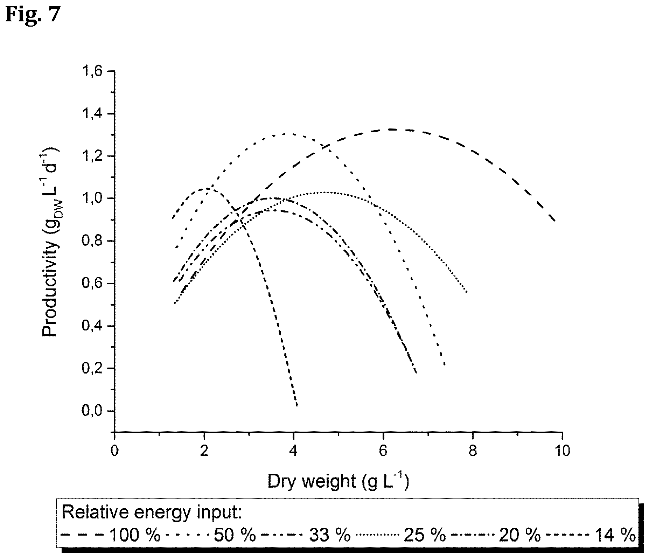

Fig. 7 shows the productivity of the bioreactor against dry weight. The peak depends on the amount of air introduced into the bioreactor as well as the prevailing light intensity, but it will be seen that the optimal mass concentration of the algae is around 4-5 g/l and this is the concentration at which the highest productivity takes place. In other words, the best time for extraction of the algae from the bioreactor will be just before the peak value is reached.

### Reference Numerals

- 10: Bioreactor
- 20: Rise chamber
- 25: Product outlet
- 30: Down chamber
- 40: Gas inlet
- 50: Gas pipe
- 60: Carbon dioxide pipe.
- 62: Flowmeter
- 63: Second gas inlet
- 64: Carbon dioxide valve
- 66: Carbon dioxide source
- 70: Air pipe
- 72: Air valve
- 74: Mass-flow controller
- 76: Compressor
- 78: Controller
- 79: PAR sensor
- 80: Medium inlet
- 90: Medium pipe
- 92: First medium source
- 94: First medium valve
- 96: Second medium valve
- 100: Second medium source
- 110: Outlet pipe
- 210: Wall elements
- 220: Transverse elements
- 230: Interior chambers
- 240: Channel

## Claims

1. A bioreactor (10) comprising:
at least one rise chamber (20) containing a growth medium;
at least one fall chamber (30) connected to the at least one rise chamber (20) at a top and at a bottom to form a loop;
a first gas inlet (40) connected to the at least one rise chamber for supplying a first gas flow to the at least one rise chamber (20); and
a second gas inlet (63) connected in the loop to supply a second gas flow to the loop,
the second gas flow being enriched, compared to air, with carbon dioxide.

2. The bioreactor (10) of claim 1, wherein the second gas flow is enriched with more than 10% with carbon dioxide.

3. The bioreactor (10) of claim 1 or 2, wherein the second gas inlet (63) is located at the top of or in the at least one fall chamber (30).

4. The bioreactor (10) of any of the above claims, wherein the second gas is supplied substantially bubble free in the growth medium.

5. The bioreactor (10) of any of the above claims, wherein the carbon dioxide gas is supplied to the bottom of the at least one rise chamber (20).

6. The bioreactor (10) of any of the above claims further comprising a controller for controlling the flow rate of the second gas flow.

7. A method of operation of a bioreactor comprising:
supplying the bioreactor (10) with a growth medium for growing at least one of phototrophic microorganisms or phototrophically grown cells;
supplying the bioreactor (10) with a first gas flow to intermix the growth medium, the growth medium including at least one of the phototrophic microorganisms or the phototrophically grown cells; and
supplying the bioreactor (10) with a second gas flow, the second gas flow being, compared to air, enriched with carbon dioxide.

8. The method of claim 7, wherein the supplying of the carbon dioxide gas is substantially bubble-free

9. Use of the bioreactor according to any one of the claims 1 to 7 to grow phototrophic microorganisms or phototrophically grown cells.

10. The use of claim 9, wherein the phototrophic microorganisms are at least one of algae or cyanobacteria.
